# EUROPEAN PATENT APPLICATION

(11) **EP 2 884 283 A1**
(43) Date of publication of application: **17.06.2015**
(21) Application number: 13196554.3
(22) Date of filing: 10.12.2013
(51) Int. Cl.: G01N 33/70, G06F 19/00

(54) **A method for predicting delayed graft function in kidney transplantation**

(71) Applicant: Chu Nantes, 44000 Nantes (FR); Assistance Publique - Hôpitaux de Paris, 75004 Paris (FR); Université Paris Descartes, 75006 Paris (FR); Université de Nantes, 44000 Nantes (FR); Institut National de la Santé et de la Recherche Médicale (INSERM), 75013 Paris (FR); Imagine Institut des Maladies Génétiques Necker Enfants Malades, 75015 Paris (FR)
(72) Inventor: Foucher, Yohann, 44000 Nantes (FR); Giral, Magali, 44470 Carquefou (FR); Daguin, Pascal, 44470 Nantes (FR); Leborgne, Florent, 44200 Nantes (FR); Chapal, Marion, 44100 Nantes (FR); Legendre, Christophe, 75011 Paris (FR)
(74) Representative: Regimbeau

(57) **Abstract**

The present invention relates to a method of predicting delayed graft function (DGF) after kidney transplantation in patient, said method comprising a step of calculating a score (DGFS) based on the following variables: induction therapy, Cold ischemia time (CIT), donor creatinin levels, donor age, and patient BMI. Methods for assessing the need for a DGF therapy after kidney transplantation are disclosed. A processing system including a computation unit and an input interface, characterized in that said system includes means for implementing the method for determining the risk of DGF is also provided.

## Description

### Introduction

Within the past decade, whilst the frequency of acute allograft rejection episodes has dramatically decreased under modern immunosuppressive regimens (1), the incidence and severity of delayed graft function (DGF) has remained stable. DGF is clinically defined by the need for dialysis within the first seven days post transplantation for non living heart-beating donor grafts (2), with an incidence ranging from 25 to 50% (3). This large variability is mainly explained by the lack of one single DGF definition (2). Another possibility is the more widespread use of Expanded Criteria Donors (ECDs) or non heart-beating donors in order to limit the duration on organ waiting lists (3). DGF is known to impair one-year post transplantation renal function and to decrease long-term graft and recipient survival. DGF also has significant economic consequences, with more prolonged hospitalization, increased patient management costs, and the need for dialysis, diagnostic radiology, needle core biopsies and closer immunosuppressive drug monitoring (4, 5).

Preventing DGF by controlling risk factors related to both donors and recipients are among the most beneficial strategies. These include, for example, avoiding donors with hydroxyethylstarch colloid after brain death, or the preferential use of dopamine for donor resuscitation (6, 7). The further development and pre-clinical testing of new drugs, that show promising results in animal studies to prevent ischemia-reperfusion injury, could also be useful in preventing DGF (8-11). As suggested by previous studies, the use of Anti-Thymocyte Globulins as induction therapy represents a possible treatment for reducing the prevalence of DGF (12-14), however others have questioned this (15-18). In order to increase its efficacy, depleting induction therapy should be prescribed only for patients at-risk of DGF. Therefore, accurately predicting DGF appears very important in kidney transplantation management.

Several DGF scoring systems have been proposed within the last few years. First published in 2003 by Irish et al (19) and then refined in 2010 (20), the authors proposed a predictive model by using donor and recipient characteristics known at the time of transplantation from the United State Renal Data System (USRDS) registry. They proposed a predictive score that could be calculated at the time of transplantation, which was validated by using an independent data associated with an area under the Receiver Operating Characteristic Curve (AUC) at 0.70. Despite the high quality of this methodology, this predictive model has several limitations. Firstly, this score was developed and validated from North American recipients, whereby the immunosuppressive therapy (dosage, induction, etc), the definition of adverse events and the patients' profiles differ substantially from European recipients (21, 22). Secondly, its use in clinical practice may be problematic due to the high number of variables and the lack of a decision threshold aimed at classifying patients according to their DGF risk.

Jeldres et al. (23) proposed a simpler but equally accurate scoring system (six variables, AUC=0.74), however these results were based on a monocentric study of North American recipients transplanted since 1979, allowing interrogation on its accuracy for recent transplant in other regions.

More importantly and for both available scoring systems (20, 23), the recipients were included regardless of their induction therapy as this was not studied as a parameter of the scoring system. It is thus difficult to use these scores for better management of the induction therapy.

There is thus still a need for a method for predicting DGF which would be universal and which could be used easily in clinic.

### Description

The present inventors have now developed a new method for assessing the risk of DGF in renal transplantation. In particular, they have developed a complementary DGF Score, tentatively called DGFS, from a French multicentric and prospective cohort of kidney transplant recipients since 2007.

Previous scoring systems had been developed in the prior art, especially regarding the biostatistics methodology for variables selection. However, none of them took into account the induction therapy, which remains one of the possible interventions available clinically to potentially decrease the occurrence of DGF. In contrast, the DGFS includes induction therapy as one of the variables useful for its computation. In addition, the inventors have shown that four other independent variables, namely cold ischemia time (CIT), donor age, donor creatinine levels, and recipient BMI, contributed significantly to the DGF prognostic.

The DGFS comprises only these five variables, a clear advantage compared to the scoring system developed by Irish. These five variables are easily available at the time of transplantation, which may not be the case for the 18 of the Irish's scoring system. In particular, the donor weight is difficult to evaluate for deceased donors, warm ischemia time is not systemically measured by surgeons, and some national laws may prevent the donor ethnicity from being collected.

Importantly, the DGFS shows good predictive capacities, at least as good as the one of the scoring systems of the prior art. In particular, the DGFS, although much simpler than the system developed by Irish (5 variables instead of 18), is as predictive. Moreover, the DGFS outperformed the six-parameter scoring system proposed by Jeldres et al.

Thus, in a first aspect, the invention provides a method for predicting delayed graft function (DGF) after kidney transplantation in patient, said method comprising the steps of:
a) determining the following variables:
   - induction therapy,
   - Cold ischemia time (CIT),
   - donor creatinine levels,
   - donor age, and
   - patient BMI;
b) calculating a score (DGFS) by computing the variables of step a), and
c) determining the risk of DGF in view of the DGFS.

A kidney transplantation (or renal transplantation) is a surgical procedure performed to replace a diseased kidney in a patient, the recipient, with a healthy kidney from another person, the donor. A kidney transplant may be recommended for people with end stage renal disease (ESRD), a permanent condition of kidney failure that often requires dialysis (a process used to remove wastes and other substances from the blood). Examples of diseases which may result in ESRD include, but are not limited to, the following: repeated urinary infections, kidney failure caused by diabetes or high blood pressure, polycystic kidney disease or other inherited disorders, glomerulonephritis, hemolytic uremic syndrome, lupus and other diseases of the immune system, obstructions. Other conditions, such as congenital defects of the kidneys, may also result in the need for kidney transplantation.

The kidney which is grafted may come from a deceased organ donor or from a living donor. Preferably, the donor of the invention is a deceased person (cadaver). Deceased donors include both heart-beating and non-heart-beating donors. A "heart-beating donor" according to the invention is an individual whose death resulted from irreversible damage to the brainstem. A "non-heart-beating donor" according to the invention is an individual whose death has been determined by cessation of heart and respiratory function rather than loss of whole brain function. Preferably, the donor according to the method of the invention is a heart-beating donor.

Within the scope of the present invention, "delayed graft function" or DGF is defined as the medical condition after organ transplantation characterized by the absence or incompleteness of the respective organs functions compared to a transplant not suffering from this condition. As used herein, "DGF" refers to the need for the dialysis within the first seven days pot-transplantation, which is the most common definition.

By "cold ischemia time" or "CIT", it is herein referred to the time between the chilling of a tissue, organ, or body part after its blood supply has been reduced or cut off and the time it is warmed by having its blood supply restored. This can occur while the organ is still in the body or after it is removed from the body if the organ is to be used for transplantation.

It is known in the art that, generally, the shorter this time, the more likely the kidney is to work immediately and the better the long-term outcome. Indeed, the present inventors have shown that the probability of DGF increased with the CIT: for example, an increase of six hours was associated with a 1.42-fold risk of DGF.

"Creatinine" as used herein refers to (2-amino-1-methyl-4-imidazolidinone) is a stable, natural end-product of creatine (also known as N-(aminoiminomethyl)-N-methylglycine; mefhylglycosamine or N-methyl-guanido acetic acid) catabolism in muscle tissue. Creatinine is made at a steady rate and is not affected by diet or by normal physical activities. It is present in serum and in urine at approximately 100 µM concentrations. As is well known to the person of skills in the art, if the kidneys are damaged and cannot work normally, the amount of creatinine in the urine goes down while its level in the blood goes up.

Tests for measuring creatinine levels have been widely used in the clinic for the last century. In fact, creatinine levels tests are routinely performed by laboratories all around the world (see for a review e.g. reference 50). Such methods need not be further detailed here.

Creatinine levels according to the invention are measured in a biological sample from the donor. A "biological sample" may be any sample that may be taken from the donor. Such a sample must allow for the determination of the creatinine levels of the invention. Preferred biological samples for the determination of the said creatinine levels include samples such as a blood sample, a plasma sample, or a lymph sample. Preferably, the biological sample is a blood sample. More preferably, this blood sample is collected at the time of the death of the donor.

The inventors have shown that donor creatinine levels lower than 108 µmole.L⁻¹ were significantly associated with lower risks of DGF. Thus in a preferred embodiment, the calculation of the DGFS according to the invention involves determining whether the donor creatinine levels lower or higher than 108 µmole.L⁻¹.

The "body-mass index" (BMI) is a simple index of weight-for-height that is commonly used to classify underweight, overweight and obesity in adults. It is commonly defined as the weight in kilograms divided by the square of the height in meters (kg/m²).

The "donor age" according to the invention is preferably the donor age at the time of death, expressed in years.

An "induction therapy" according to the invention refers to a therapy providing a high degree of immunosuppression at the time of transplantation, thus preventing acute rejection during the early post-transplantation period. It is known that the risk of acute rejection of the graft is maximal in the few weeks or months following the transplantation. The induction therapy of the invention is more specifically the absence or the presence of a strong initial immunosuppressive therapy which depletes or modulates B- or T-cell responses, thus leading to rapid, specific, short term-modulation of the immune system (51). Induction therapy is often considered essential to optimize outcomes, particularly in patients at high risk for poor short-term outcomes.

The induction therapy according to the invention comprises the administration to the patient of an induction agent. An "induction agent" according to the invention means any agent used in an induction therapy of the invention. More specifically, an induction agent is any agent used perioperitatively which re-establishes or induces peripheral immune tolerance in the context of transplant rejection. Most of the induction immunosuppressive agents currently used are biological agents and are either monoclonal (muromonab-CD3, daclizumab, basiliximab, alemtuzumab) or polyclonal (antithymocyte globulin [equine] or antithymocyte globulin [rabbit]) antibodies.

Induction agents can be either non-depletive agents or depletive agents.

Non-depletive agents are induction agents which modulate T-cell responses without affecting the titer of circulating lymphocytes. For example, basiliximab (Simulect®) and daclizumab (Zenapax®) are both monoclonal antibodies which bind the α chain of the interleukin 2 receptor (IL2R). They competitively inhibit the binding of interleukin 2 (IL2) to the receptor, thus inhibiting IL2-driven proliferation of activated T-cells.

These antibodies are increasingly used as inducing agents because they show very little side effects (52). Their excellent tolerability and safety profile indeed make IL2R α chain-blocking antibodies an attractive regimen for such an induction therapy.

Depletive agents result in the depletion of circulating lymphocytes, for example by causing T-cell lysis and/or clearance. Examples of such depleting agents include antithymocyte globulin (ATG), muromonab-CD3 (trade name Orthoclone OKT3, marketed by Janssen-Cilag) and alemtuzumab (commercialized under the names Campath®, MabCampath®, Campath-1H® and, more recently, Lemtrada®).

Alemtuzumab is a humanized monoclonal antibody directed against CD52, a glycoprotein present on virtually all B- and T-cells as well as macrophages, NK cells, and some granulocytes. The binding of alemtuzumab to its cognate CD52 target triggers activation of the Antibody-Dependent Cell-Mediated Cytotoxicity (ADCC) mechanism, thus triggering lysis of the cell.

Muromonab-CD3 is a murine monoclonal antibody directed against the CD3 receptor part of the T-cell receptor/CD3 complex (TCR/CD3 complex) and involved in T-cell development and signal transduction. Muromonab-CD3 binding to CD3 leads to endocytosis of the TCR, resulting in an inert T-cell. Such T-cells are then removed via opsonization and ultimately phagocytosis.

ATG is an infusion of horse or rabbit-derived polyclonal antibodies against human T cells, which is used in the prevention and treatment of acute rejection in organ transplantation and therapy of aplastic anemia. ATG is commercially available from several drug companies, such as Fresenius (ATG-Fresenius®), Genzyme (Thymoglobulin®), and Pfizer (Atgam®).

ATG administration very substantially reduces immune competence in patients with normal immune systems, through a combination of actions. More specifically, ATG targets a wide range of T-cell surface antigens as well as natural killer-cell antigens, B-cell antigens, plasma cell antigens, adhesion molecules and chemokine receptors, resulting in profound, long-lasting T-cell depletion (53).

However, ATG has many side effects, some serious and others very uncomfortable. Most of these side effects occur because ATG itself is made up of horse or rabbit proteins, which are recognized as foreign by the recipient's immune system. For example, ATG can cause serious allergic reactions with symptoms including skin rashes, low blood pressure, and problems breathing. It has even been reported about 15% of patients develop leukemia or myelodysplasia several years after getting ATG. It is thus important to ensure that ATG administration is reserved to cases that warrant it.

The present inventors have shown that the nature of the induction agent (ATG vs. IL2R antibodies or no induction) reflects strongly on the risk of developing DGF. A lower risk of DGF was observed when induction therapy comprised a step of administering ATG, compared with an induction therapy which does not comprise such a step.

Thus, in a preferred embodiment the induction agent used in the induction therapy of the method of the invention is ATG. In another preferred embodiment, the said induction agent is an antibody directed against IL2R. In yet another preferred embodiment, no induction agent is administered. In a further preferred embodiment, the induction therapy comprises the administration of an antibody directed against IL2R or of no induction agent.

According to a preferred embodiment of the method of the invention, the DGF Score (DGFS) is calculated by:
1) multiplying the logarithm of the odds ratio by the value of the corresponding variables,
2) summing up the five previous numbers,
3) subtracting 3.546 to the previous number and
4) dividing the previous number by 0.626.

The values of the variables correspond to the measured CIT (expressed in hours), donor age (expressed in years) and patient BMI (expressed in kg/m²).

For the induction therapy and the creatinine levels, the values are 1 if the patient had no ATG induction therapy or if creatinine levels are higher than 108 µmole.L⁻¹, respectively. Conversely, the values are 0 if the patient had been administered ATG as an induction agent or if creatinine levels are lower than or equal to 108 µmole.L⁻¹.

The odds ratio (OR) evaluates whether the odds of a certain event or outcome is the same for two groups (see e.g. 54). Specifically, the OR measures the ratio of the odds that an event or result will occur to the odds of the event not happening. In the present case, an OR is a measure of association between any one of the variable of the DGFS (induction therapy, CIT, donor age, donor creatinine levels, and recipient BMI) and an outcome of DGF.

The OR is one of several statistics that have become increasingly important in clinical research and decision-making. It is particularly useful because as an effect-size statistic, it gives clear and direct information to clinicians about which treatment approach has the best odds of benefiting the patient. In the method of the invention, the following ORs were estimated by maximization of the likelihood of the logistic model:
- OR = 1.06 for CIT (p < 0.0001), 95%CI=[1.04, 1.08];
- OR = 1.70 for no ATG (p = 0.0001), 95%CI=[1.30, 2.23];
- OR = 1.76 for donor creatinine levels > 108 µmole.L⁻¹ (p = 0.0004), 95%CI=[1.29, 2.41];
- OR = 1.06 for BMI (p = 0.0004), 95%CI=[1.02, 1.09]; and
- OR = 1.02 for donor age in years (p = 0.0004), 95%CI=[1.04, 1.08][1.01, 1.02].

The risk of DGF increases with the DGFS value. The DGFS offers useful advantages in terms of interpretations, since this score follows a standard normal distribution (mean at 0 and standard deviation at 1). A patient with a positive DGFS can be considered at risk of DGF higher than the mean risk. In contrast, a patient with a negative score can be considered at risk of DGF lower than the mean. About 70% of individuals are within one standard deviation, i.e. with a score value between -1 and 1. About 95% of individuals are within two standard deviations, i.e. with a score value between -2 and 2.

It is thus particularly useful for assisting clinicians in predicting the occurrence of DGF, and thus making better informed treatment and allocation decisions.

In this regard, it is particularly important to provide thresholds for the clinicians for clinical intervention decision making. Such thresholds should indicate to the clinicians whether the patient has a low or high risk of DGF with a high level of confidence.

It has been observed that the percentage of patients without DGF was around 88% if the DGFS was lower than -0.50. For patients with a DGFS lower than this threshold, clinicians have a 12% chance to incorrectly predict DGF. Thus, one could use this threshold with a good confidence in order to identify patients with a low-risk of DGF (32% of recipients had a DGFS lower than -0.50). Therefore, in an embodiment of the invention, the risk of DGF is considered to be low if the DGFS is inferior or equal to - 0.50.

In contrast, the percentage of patients with DGF was found to be around 50% if the DGFS was higher than 1.20. This threshold could be used in order to identify high-risk patients (11% of the patients had a DGFS higher than 1.20). In another embodiment of the invention, the risk of DGF is estimated to be high if the DGFS is higher than 1.20.

As a consequence, patients with a DGFS between -0.5 and 1.20 can be considered at medium risk. In yet another embodiment of the invention, the risk of DGF is considered to be medium if the DGFS is comprised between -0.50 and 1.20.

Such thresholds are relevant clinically, since they help the clinicians make decisions about whether the patient is at risk of DGF and whether this risk should be treated in consequence. In particular, the DGFS makes it possible for the clinician to predict whether the patient is at risk of DGF before the kidney transplantation and to administer a therapy for this DGF. Notably, it makes it possible for the clinician to adapt the induction therapy in respect of the DGFS before the actual kidney transplantation takes place.

Thus, in another aspect, the invention is drawn to a method for deciding for assessing the need of a DGF therapy for a kidney transplantation, said method comprising the steps of:
a) determining the risk of DGF according to the method of the invention, and
b) administering a therapy after the kidney transplantation if the DGFS is above a threshold.

"DGF therapy" as used herein includes all the treatment commonly used to alleviate the symptoms of DGF and, in particular, to prevent any detrimental effects for both graft and patient survival. In particular, the determination of the DGFS may for instance help to decide whether ATG should be administered to the patient. The inventors have shown that induction therapy with ATG was correlated with the DGF risk regardless of the value of CIT and other confounding factors, in contrast to other scoring systems. Hence, it is a particularly useful tool for clinicians to assist in medical decision making to decrease the risk of DGF by using a depleting induction therapy. Such DGFS use has to be validated by a future clinical trial.

Therefore, in a specific embodiment of the method of the invention, the DGF therapy is an induction therapy with ATG as the induction agent.

In this respect, it may be advantageous, in order to decide whether an induction therapy with ATG might be beneficial, to determine the DGFS for the patient with no induction or with an induction by an IL2R antibody. If the calculated DGFS is lower than -0.50, then the risk of DGF is very low, and there is thus no reason to embark on an ATG therapy, especially in view of the associated side effects. If the calculated DGFS is higher than 1.20, then the risk of DGF is high, and ATG should be prescribed.

More advantageously, the DGFS calculated for the patient with no induction or with an induction by an IL2R antibody maybe compared to the DGFS calculated for the same patient under ATG therapy. In particular, when the calculated DGFS with no induction or with an induction by an anti-IL2R antibody is between -0.50 and 1.20, such a comparison is particularly useful for deciding whether ATG administration would be advantageous. Indeed, if the DGFS calculated with the ATG induction is superior or equal to the DGFS calculated for the patient with no induction or with an induction by an IL2R antibody, the benefit of the ATG therapy is probably irrelevant in regard of the corresponding adverse events. On the other hand, if the DGFS calculated with the ATG induction is inferior to the DGFS calculated for the patient with no induction or with an induction by an IL2R antibody, then it would be advantageous to administrate ATG as induction agent to the patient.

According to this specific embodiment, the method of the invention thus comprises the steps of:
a) determining the risk of DGF for the patient with ATG induction according to the method of the invention,
b) determining the risk of DGF for the said patient with no induction or with an induction by an IL2R antibody according to the method of the invention, and
c) administering a therapy if the DGFS calculated in step a) is inferior to the DGFS calculated in step b).

The invention also relates to a product/computer program containing a set of instructions characteristic of the implementation of the inventive method.

The invention also relates to a processing system including a computation unit and an input interface, characterized in that said system includes means for implementing the method for determining the risk of DGF as disclosed herein.

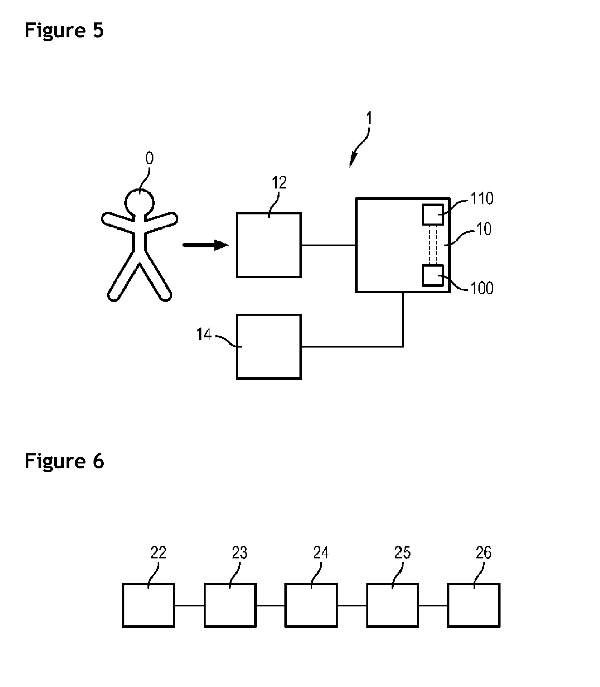

In reference to Figure 5, a device (1) according to a particular embodiment of the present invention includes a computation unit (10) capable of following computer instructions and processing data. One such computation unit preferentially includes a microprocessor (110) which can be of any type known in the state of the art. The computation unit (10) also has a storage unit (100) that is capable of receiving a computer program including a set of instructions characteristic of the implementation of the method, and is capable of storing data.

The device (1) also includes an input interface (12) connected to the computation unit (10) enabling an operator (O) of the device (1) to enter data to be treated. One such input interface (12) includes any element enabling the entry of such data destined for the computation unit (10) such as a keyboard element optionally associated with a pointing device element.

Preferentially, the computation unit further includes an output interface (14) such as a screen that on the one hand enables the user to verify the integrity of the data entered but on the other hand enables the computation unit (10) to be able to interact with the operator (O).

The device (1) can be integrated in a single system such as a computer, a smartphone or any other system known in the state of the art enabling implementation of the inventive method. The operator (O) can be of any skill level and thus may or may not have medical qualifications.

It is notably envisaged according to a particular embodiment of the present invention that the data entered by the operator (O) are sent via a network (the Internet, for example) preferentially in a secure manner to a remote server comprising a computation unit capable of implementing the inventive method and thus of treating the data received by the server. Optionally, after said processing, the server returns the result of the analysis to the user via the same network or another. Optionally, the server records the data and/or the result of the analysis on a means of recording. Obviously, means of guaranteeing the anonymity of the physiological/clinical characteristics of the donor and the recipient can be envisaged.

Thus, one such device (1) enables implementation of the inventive method, i.e., it enables implementation of the following steps:
- entering physiological/clinical characteristicsusing the input interface (12) into a computation unit (10) (step 22), said characteristics including:
   - induction therapy,
   - Cold ischemia time (CIT),
   - donor creatinine levels,
   - donor age, and
   - patient BMI;
- determining the variables on the basis of said characteristics via data processing by the computation unit (10) (step 23),
- combining said variables so as to obtain the DGFS via data processing by the computation unit (10) (step 24), and
- analyzing said risk score so as to determine a DGF risk (step 25).

Thus, the method of the invention can be implemented not only by clinical or hospital personnel but also by all persons involved in clinical research (pharmaceutical industry, scientists, doctors, etc.).

The examples that follow are merely exemplary of the scope of this invention and content of this disclosure. One skilled in the art can devise and construct numerous modifications to the examples listed below without departing from the scope of this invention.

### Figure legends

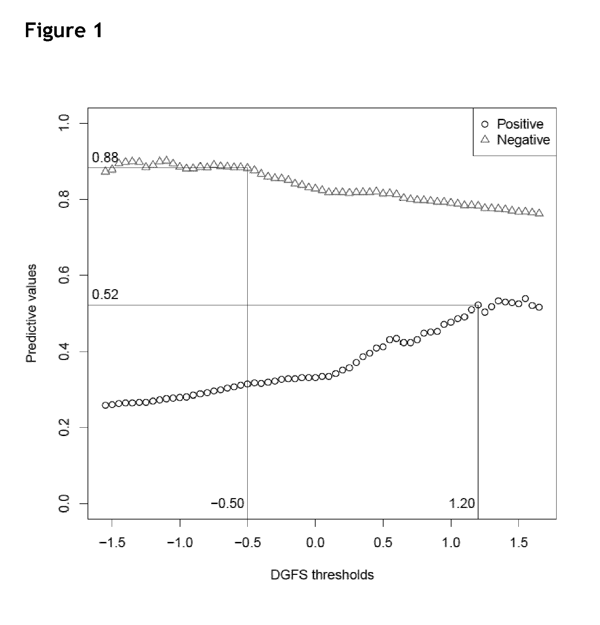

**Figure 1****:** Positive and negative predictive values according to the threshold values. These analyses were performed from the training sample (n = 1222, 16 observations removed because of missing data). If the score is higher than the threshold, the test is considered positive and the patient at risk of DGF. In contrast, if the score is lower than the threshold, the test is negative and the patient is not considered at risk of DGF. The percentage of patients with DGF was around 50% if the DGFS was higher than 1.20. The percentage of patients without DGF was around 88% if the DGFS was lower than -0.50.
**Figure 2****:** Receiver Operator characteristic (ROC) curves to evaluate the prognostic capacity of the different markers performed from the validation sample. The composite score (red continuous line, n = 561, AUC=0.73, 95% CI = [0.68, 0.77]). The cold ischemia time (black dashed line, n = 561, AUC=0.64, 95% CI = [0.59, 0.69]). The score proposed by Jeldres et al. (blue dotted line, n = 226, AUC=0.64, 95% CI = [0.56, 0.72]).
**Figure 3****:** Evaluation of the calibration of the model. The observed and predicted DGF probabilities were computed according to 10 DGFS intervals. The goodness of the fit cannot be rejected (p=0.1054, Hosmer-Lemeshow statistic).
**Figure 4****:** Patient and graft survival according to the DGFS from the validation sample (N = 568) using the Kaplan-Meier estimator.
**Figure 5****:** Schematic representation of a processing system according to a particular embodiment of the present invention
**Figure 6****:** Functional graph representing a method according to a particular embodiment of the present invention

### Experimental examples

### Patients and Methods

### Studied population

Adult (≥ 18 years) recipients of isolated, non pre-emptive, non machine-perfused, deceased donor kidneys, who were prospectively collected since January 2007 and computerized in the DIVAT (Données Informatisées et VAlidées en Transplantation, www.divat.fr, N° CNIL 891735 version 2, August 2004) data bank were included in the study. Biological and clinical data within DIVAT are prospectively recorded according to a common thesaurus at participating centers (since 1996 for Nantes, Paris Necker, Montpellier and Nancy, since 2003 for Toulouse and since 2007 for Lyon). Codes were used to ensure donor and recipient anonymity and blinded testing. The data are computerized in real time as well as at each transplant anniversary. The quality of the DIVAT data bank is validated by an annual cross-center audit, systematic verification during data entry, and a weekly automatic report on the identification of incoherencies between parameters.

The following classes of renal allograft donor organs or recipients were not included in the study : kidney transplantations from living donors and non heart-beating donors; preemptive transplantations; the use of pulsatile perfusion machine for graft's preservation having an impact on early graft function; patients treated by peritoneal dialysis before transplantation (peritoneal dialysis often used after transplantation for patient comfort and therefore not a witness of DGF) and patients who displayed less than seven days of patient and-graft survival (seven days being necessary to diagnose DGF). Moreover, we excluded patients with at least one missing value for cold ischemia time (CIT), recipient's gender, HLA incompatibilities and PRA because these are considered as major risk factors within the DGF literature.

One thousand eight hundred and forty four adult renal transplant recipients were finally included in the study, among whom 468 (25.4%) experienced DGF, as defined by the need for dialysis within the first seven days post transplantation. The standard immunosuppression regimen for renal transplants from cadaveric donors partly varied with the number of previous transplants and high or low immunologic risk. This regimen consisted of induction therapy with corticosteroids and anti thymocyte glogulin (ATG) from the time of surgery for all recipients with a high immunological risk. In recipients with a low immunological risk, anti-interleukin 2 receptor antibodies (anti IL2R) or no induction was generally prescribed, except in case of high prolonged CIT or ECD, where ATG was mostly prescribed in addition to calcineurin inhibitors either from day 0, or after a short delay (between two and five days) depending of centre habits. All patients received a maintenance immunosuppression combination of calcineurin inhibitors (Tacrolimus, Cyclosporine) with Mycophenolate mofetil. These two drugs were eventually associated with Prednisone, progressively tapered to 5 to 10 mg per day, or stopped within the first three months of follow-up except for specific indications (acute rejection, re-transplantation, high immunological risk or Mycofenolate Acid adverse events).

### Available data

The DGF is defined as the need for dialysis within the first seven days after the renal transplantation. The following recipient variables were extracted from the database at the time of transplantation: age, gender, body mass index (BMI), time on dialysis before transplantation, previous transplants, initial kidney disease (differentiating recurrent graft diseases from others diseases), induction immunosuppressive treatment differentiating the use of ATG from the use of either anti IL2R or no induction (no ATG), anti HLA class I or II PRA positivity (> 0%) and past histories of diabetes mellitus, high blood pressure, cardiovascular events, dyslipidemia, B hepatitis and neoplasia. Extracted donor variables were: age, gender, primary cause of death, type of vasopressor employed (Dobutamine, Dopamine, Epinephrine or Norepinephrine) and terminal serum creatinine. Transplant variables were: CIT and number of HLA-A, -B, -DR incompatibilities.

### Statistical analyses

The entire sample data was randomly assigned into two groups in a proportion of two-thirds (n = 1 238) for a learning sample and one-third (n = 606) for a validation sample.

Comparisons of these two samples confirmed their homogeneity (see tables 1 and 2).

*(i) Construction of the scoring system -* This analysis was performed based on the learning sample by using a logistic regression to study the DGF probability. Univariate analyses were performed for the validation of log linearity assumptions associated with continuous variables (B-spline transformation), and for a first selection of explicative variables (p-value < 0.20). If the log linearity did not hold, the variables were categorized according to clinically relevant thresholds.

The variables were further analysed in a multivariate model and retained if their corresponding p-value was lower than 0.05. Some interactions between explicative variables were tested according to the literature and relevant clinical interpretations.

Finally, the last significant variables were removed if the corresponding decrease of the area under ROC curve (AUC) was lower than 1%. The scoring system was the linear predictor of this final model. For a better interpretation, we normalized this score by subtracting the mean and dividing by the standard deviation in order to obtain the DGFS.

*(ii) Validation of the scoring system -* The model was applied to the independent data set and the corresponding ROC curve was calculated for validation. 95% confidence intervals (95% CI) of the AUC were non-parametrically obtained by bootstrap resampling (1000 iterations).

The calibration of the model was also evaluated, i.e. the concordance between the observed DGF probabilities and the expected ones. Ten intervals of the DGFS were used for the corresponding plot and Hosmer-Lemeshow statistic (p < 0.05 indicates a rejection of the goodness-of-fit). All the analyses were performed using R version 2.15 (24).

*(iii) Graft and patient survival analysis -* The outcome was defined as the post transplantation time until the first event being either death with functioning graft or return to dialysis. The Kaplan-Meier estimator was used for calculating the survival probabilities and the Cox model for computing the hazard ratio (HR). The differences were tested by using the logRank statistic.

### Results

### Donor, Recipient and Renal Transplant Characteristics

Qualitative and quantitative variables are described according to the training and validation samples in tables 1 and 2.

**Table 1. Recipient, donor and kidney transplant continuous characteristics (minimum, maximum, mean and standard deviation) at time of transplantation according to both learning and validation samples.**

| | **learning (n = 1238)** | | | | **validation (n = 606)** | | | |
|---|---|---|---|---|---|---|---|---|
| | **min** | **max** | **mean** | **SD** | **min** | **max** | **mean** | **SD** |
| **Cold ischemia time** (hours) | 6.00 | 58.62 | 19.21 | 7.02 | 6.33 | 47.30 | 18.99 | 6.80 |
| **Recipient age** (years) | 19.00 | 84.00 | 51.92 | 13.19 | 19.00 | 84.00 | 51.96 | 12.77 |
| **Donor age** (years) | 4.00 | 88.00 | 51.91 | 15.60 | 1.00 | 90.00 | 52.92 | 40.72 |
| **BMI** (kg/m²) | 14.20 | 46.57 | 24.07 | 4.44 | 14.87 | 38.58 | 24.23 | 4.16 |
| **Donor serum creatinine** (µmol/L) | 20.00 | 999.00 | 91.02 | 60.67 | 20.00 | 335.00 | 86.39 | 44.93 |
| **Duration in dialysis** (years) | 0.02 | 36.60 | 4.07 | 3.92 | 0.04 | 34.02 | 3.79 | 3.64 |

**Table 2. Recipient, donor and kidney transplant continuous characteristics (effective and percentage) at time of transplantation according to both learning and validation samples.**

| | **learning (n = 1238)** | | **validation (n = 606)** | |
|---|---|---|---|---|
| | **effective** | **percent. (%)** | **effective** | **percent. (%)** |
| **Recipient gender** | | | | |
| male | 748 | 60.4 | 384 | 63.4 |
| female | 490 | 39.6 | 222 | 36.6 |
| **Donor gender** | | | | |
| male | 720 | 58.6 | 381 | 62.9 |
| female | 509 | 41.4 | 225 | 37.1 |

| **Rank of the kidney transplantation** | | | | |
|---|---|---|---|---|
| first | 927 | 74.9 | 464 | 76.6 |
| second | 255 | 20.6 | 120 | 19.8 |
| third or more | 56 | 4.5 | 22 | 3.6 |

| **Donor cause of death** | | | | |
|---|---|---|---|---|
| vascular causes | 715 | 57.9 | 343 | 56.6 |
| others causes | 520 | 42.1 | 263 | 43.4 |

| **Immunosuppressive induction therapy** | | | | |
|---|---|---|---|---|
| ATG | 684 | 55.3 | 335 | 55.3 |
| No ATG | 554 | 44.7 | 271 | 44.7 |

| **History of diabetes** | | | | |
|---|---|---|---|---|
| yes | 157 | 12.7 | 82 | 13.5 |
| no | 1081 | 87.3 | 524 | 86.5 |

| **History of hypertension** | | | | |
|---|---|---|---|---|
| yes | 1000 | 80.8 | 483 | 79.7 |
| no | 238 | 19.2 | 123 | 20.3 |

| **History of cardiovascular events** | | | | |
|---|---|---|---|---|
| yes | 448 | 36.2 | 232 | 38.3 |
| no | 790 | 63.8 | 374 | 61.7 |

| **History of dyslipidemia** | | | | |
|---|---|---|---|---|
| yes | 384 | 31.0 | 182 | 30.0 |
| no | 854 | 69.0 | 424 | 70.0 |

| **History of hepatitis B** | | | | |
|---|---|---|---|---|
| yes | 81 | 6.5 | 34 | 5.6 |
| no | 1157 | 93.5 | 572 | 94.4 |

| **History of neoplasia** | | | | |
|---|---|---|---|---|
| yes | 123 | 9.9 | 74 | 12.2 |
| no | 1115 | 90.1 | 532 | 87.8 |

| **Anti-class I PRA** | | | | |
|---|---|---|---|---|
| detectable | 468 | 37.8 | 225 | 37.1 |
| undetectable | 770 | 62.2 | 381 | 62.9 |

| **Anti-class II PRA** | | | | |
|---|---|---|---|---|
| detectable | 446 | 36.0 | 223 | 36.8 |
| undetectable | 792 | 64.0 | 383 | 63.2 |

| **Initial kidney disease** | | | | |
|---|---|---|---|---|
| with risk of recurrence on graft | 373 | 30.2 | 191 | 31.6 |
| without risk of recurrence on graft | 863 | 69.8 | 414 | 68.4 |

| **HLA Mismatches at HLA-A+B+DR** | | | | |
|---|---|---|---|---|
| 0 to 4 | 1105 | 89.3 | 548 | 90.4 |
| 5 to 6 | 133 | 10.7 | 58 | 9.6 |

Since the allocation was random, the two groups were similar. In the training sample used for the scoring system definition, the mean age of recipients was 51.9 (+/- 13.2) years and 60.4% were men. For 30.2% of the recipients, the primary indication for renal transplantation was a possible recurrent disease of the renal graft. For 20.6% and 4.5% of them, it was respectively a second transplantation and a third or more transplantation. The mean duration in dialysis before transplantation was 4.1 (+/-3.9) years.

Historical anti-HLA class I and II PRA were detectable in 37.8% and 36.0 % of the patients respectively. Equal to or more than five HLA incompatibilities on A-B-DR loci were observed in 10.7% of the recipients. The mean donor age was 51.9 (+/- 15.6) years, 58.6% were men and 57.9 % were dead because of a vascular cause. The mean terminal serum creatinine was 91.0 (+/- 60.7) µmol/L. The mean duration of CIT was 19.2 (+/- 7.0) hours.

### Construction of the DGF predictive score

In the univariate analyses detailed in the web supplementary data (table S1), possible risk factors of DGF (p <0.20) were identified: donor serum creatinine, recipient age, duration of dialysis before transplantation, donor gender, number of previous transplants, immunosuppressive induction therapy, history of cardiovascular events (except HTA) and dyslipidemia, donor treatment with epinephrine and duration of CIT, donor age and recipient BMI. Without taking into account the other risk factors, the CIT was the main predictor of DGF. More precisely, the risk of DGF was multiplied by 1.05 for each hour (95% CI = [1.04, 1.07]). The corresponding AUC was 0.60 (95% CI = [0.56, 0.64]).

**Table S1. Results of the univariate logistic regression analyses of delayed graft function based on the learning sample (n = 1,238).**

| | **OR** | **95% CI** | **p-value** |
|---|---|---|---|
| **Cold ischemia time** (hours) | 1.05 | [1.04, 1.07] | < 0.0001 |
| **Donor age** (years) | 1.01 | [1.01, 1.02] | 0.0001 |
| **Recipient BMI** (kg/m²) | 1.06 | [1.03, 1.09] | 0.0001 |
| **Donor terminal serum creat.** (ref: < 63 µmol/l) | | | 0.0329 |
| from 63 to 81.9 µmol/l | 0.86 | [0.61, 1.22] | |
| from 82 to 107.9 µmol/l | 0.84 | [0.58, 1.23] | |
| higher than 108 µmol/l | 1.39 | [0.97, 1.98] | |
| **Recipient age** (ref: < 45 years) | | | 0.0843 |
| from 45 to 54.9 years | 1.29 | [0.89, 1.86] | |
| from 55 to 64.5 years | 1.57 | [1.11, 2.22] | |
| higher than 65 years | 1.32 | [0.89, 1.96] | |
| **Duration in dialysis before surgery** (ref: < 1.5 years) | | | 0.1608 |
| from 1.5 to 2.9 years | 1.27 | [0.88, 1.86] | |
| from 3 to 4.9 years | 1.17 | [0.79, 1.74] | |
| higher than 5 years | 1.52 | [1.05, 2.23] | |
| **Rank of the kidney transplantation** (ref: first) | | | 0.1483 |
| second | 0.75 | [0.53, 1.04] | |
| third or more | 1.24 | [0.67, 2.20] | |
| **Recipient gender** (male vs. female) | 1.04 | [0.80, 1.35] | 0.7789 |
| **Donor gender** (male vs. female) | 1.20 | [0.92, 1.57] | 0.1734 |
| **Donor cause of death** (vascular causes vs. others) | 1.06 | [0.82, 1.38] | 0.6390 |
| **Induction therapy** (non-depleting or no induction vs. depleting) | 1.62 | [1.25, 2.10] [1.25, 2.10] | 0.0003 |
| **History of diabetes** (yes vs. no) | 1.28 | [0.87, 1.84] | 0.2030 |
| **History of Hypertension** (yes vs. no) | 0.94 | [0.68, 1.31] | 0.7139 |
| **History of cardiovascular events** (yes vs. no) | 1.30 | [1.00, 1.69] | 0.0499 |
| **History of dyslipidemia** (yes vs. no) | 1.21 | [0.92, 1.58] | 0.1792 |
| **History of Hepatitis B** (yes vs. no) | 1.20 | [0.71, 1.95] | 0.4877 |
| **History of neoplasia** (yes vs. no) | 1.16 | [0.75, 1.74] | 0.5010 |
| **Detectable anti-class I PRA** (yes vs. no) | 1.19 | [0.91, 1.54] | 0.2038 |
| **Detectable anti-class II PRA** (yes vs. no) | 1.14 | [0.87, 1.48] | 0.3435 |
| **Initial kidney disease** (with vs. without risk of recurrence) | 0.95 | [0.72, 1.26] | 0.7471 |
| **Donor Dobutamine treatment** (yes vs. no) | 1.12 | [0.70, 1.77] | 0.6282 |
| **Donor Epinephrine treatment** (yes vs. no) | 1.35 | [0.99, 1.83] | 0.0584 |
| **Total HLA mismatches A+B+DR** (5-6 vs. ≤ 4) | 1.27 | [0.85, 1.88] | 0.2440 |

The results of the final model are presented in table 3.

**Table 3. Results of the final logistic regression (n = 1222, 16 observations removed because of missing data). The score is the sum of the logarithm of the odds ratios multiplied by the values of the variables. For the variables indexed by *, the values are 1 if the patient had no depleting induction or if his/her donor had a creatinemia level higher than 108 µmol/l, and 0 otherwise.**

| | **log OR (±SD)** | **OR** | **95% CI** | **p-value** |
|---|---|---|---|---|
| **Cold Ischemia time (hours)** | 0.058 (±0.010) | 1.06 | [1.04, 1.08] | <0.0001 |
| **Donor age (years)** | 0.015 (±0.005) | 1.02 | [1.01, 1.02] | 0.0014 |
| **Body Mass Index (kg/m²)** | 0.054 (±0.015) | 1.06 | [1.02, 1.09] | 0.0004 |
| **Donor creatinine level > 108 µmol/L (*)** | 0.565 (±0.160) | 1.76 | [1.29, 2.41] | 0.0004 |
| **No ATG (*)** | 0.531 (±0.138) | 1.70 | [1.30, 2.23] | 0.0001 |

Five independent explicative variables seemed significantly associated with the risk of DGF. As expected, the probability of DGF increased with the CIT (OR = 1.06, p < 0.0001). An increase of six hours was associated with a 1.42-fold risk of DGF (95% CI = [1.25, 1.58]). High recipient BMI was also associated with higher DGF probability: an increase of 5 kg/m2 was associated with a 1.31-fold risk of DGF (p = 0.0004). An increase in donor age of 10 years was associated with a risk of DGF multiplied by 1.16 (p = 0.0014). In contrast, lower risk of DGF was observed when induction therapy with Anti Thymocyte Globulin (ATG) was prescribed (OR = 1.70, p = 0.0001) compared with no ATG, and if donor creatinine levels were lower than 108 µmol/L (OR = 1.76, p = 0.0004). The induction with ATG can be analysed according to two different protocols depending on inter-center variability: delayed (n = 289) or nondelayed (n = 160) introduction of calcineurin inhibitors. We did not distinguish between these treatment protocols in the score since the corresponding risks of DGF were not significantly different (p= 0.7704).

The corresponding DGF Score (DGFS) can be calculated as follows: 1) by multiplying the logarithm of the odds ratio and the values of the explicative variables, 2) by summing the five previous numbers, 3) by subtracting 3.546 to the previous number and 4) by dividing the previous number by 0.626. For example, considering a patient with a BMI at 25 kg/m² (25*0.054), with 12 hours of CIT (12*0.058), with induction therapy based on lymphocyte depleting agent (0*0.531), with a graft from a 50-year-old donor (50*0.015) for which the last serum creatinine level was 120 µmol/L (1*0.565), the sum is 3.361 and the DGFS value is -0.296 (3.361-3.546]/0.626).

The risk of DGF increases with the DGFS value. Useful advantages in terms of interpretations are associated with the standard normal distribution of this score (mean at 0 and standard deviation at 1). A patient with a positive DGFS can be considered at risk of DGF higher than the mean risk. In contrast, a patient with a negative score can be considered at risk of DGF lower than the mean. About 70% of individuals are within one standard deviation, i.e. with a score value between -1 and 1. About 95% of individuals are within two standard deviations, i.e. with a score value between -2 and 2.

In addition to these results, it is of primary importance to propose thresholds for clinical intervention decision making. This estimation is by definition subjective since it depends on the consequent interpretations of such decision (25). Figure 1 illustrates the positive and negative predictive values regarding all the possible threshold values in order to help clinicians to define the most relevant decision depending on their context. One can also propose 2 generic decision rules. The percentage of patients without DGF was around 88% if the DGFS was lower than - 0.50. For patients with a DGFS lower than this threshold, clinicians have a 12% chance to incorrectly predict DGF. Thus, one could use this threshold with a good confidence in order to identify patients with a low-risk of DGF (32% of recipients had a DGFS lower than -0.50). In contrast, the percentage of patients with DGF was around 50% if the DGFS was higher than 1.20. One could use this threshold in order to identify high-risk patients (11% of the patients had a DGFS higher than 1.20). Importantly, there is a one in two chance to incorrectly predict DGF in patients with a DGFS higher than 1.20. As a consequence, patients with a DGFS between -0.5 and 1.20 can be considered at medium risk.

### Validation of the predictive capacity of the score

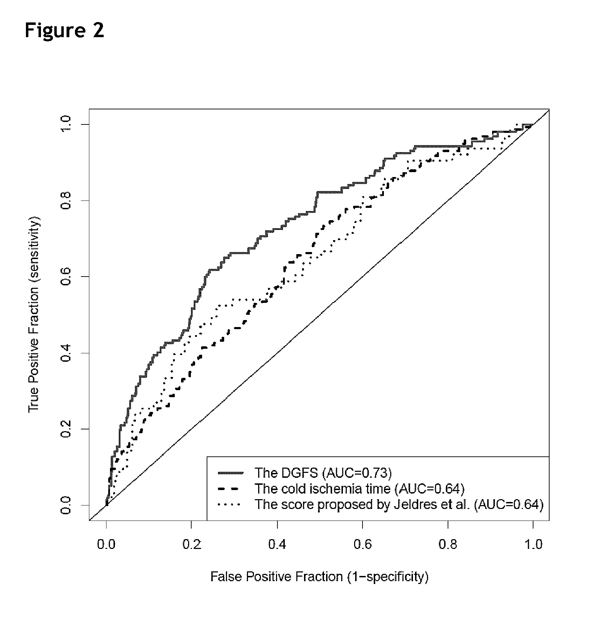

To evaluate the DGFS predictive capacity, we used this scoring system on the independent data set composed by 606 recipients. More precisely, 561 patients without missing data for the 5 parameters of the score were studied. As illustrated in figure 2, the AUCs were 0.64 (95% CI = [0.59, 0.69]) and 0.73 (95% CI = [0.68, 0.77]) for the CIT and the DGFS respectively.

The predictive capacity of the DGFS was very close to that obtained by Irish et al. (20), in which 18 parameters were included with an AUC at 0.70 (no confidence interval). These 18 parameters was not all available in our database, in particular the race (the French law does not authorize the storage of patient ethnicity) and the warm ischemia time (not always collected by surgeons). Nevertheless, we computed the score proposed by Jeldres et al. (23) based on patients without missing data on the 6 following parameters: CIT, recipient age, recipient weight, HLA mismatches, PRA and donor age. The predictive capacity of the score by Jeldres et al. seemed equivalent to a decision based only on the CIT in this validation sample (n = 226, AUC = 0.64, 95% CI = [0.56, 0.72]). The same equivalence was found from the training sample (Jeldres' Score: n = 454, AUC = 0.58, 95% CI = [0.52, 0.73]; CIT: n = 1227, AUC = 0.60, 95% CI = [0.56, 0.64]).

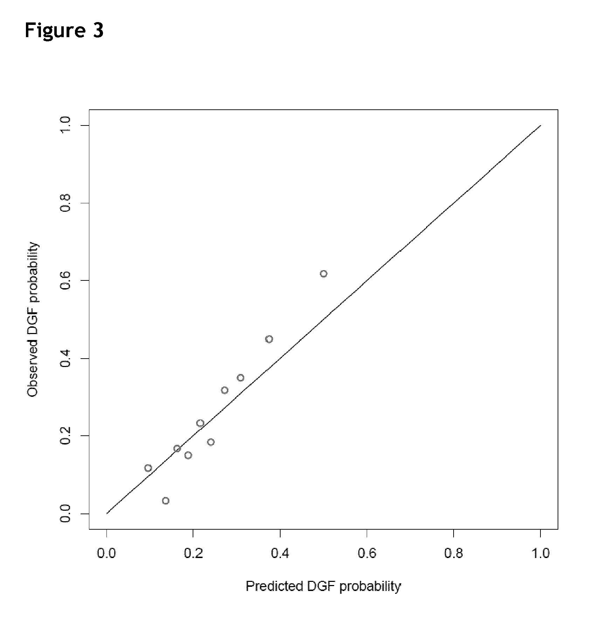

In addition, figure 3 illustrates the good calibration of the model. The predicted and observed probabilities were close regardless of the DGFS level (p=0.1054, Hosmer-Lemeshow statistic). In this validation sample, the positive predictive value of a DGFS value higher than 1.20 was 0.61 (95% CI = [0.50, 0.72]). The negative predictive value of a DGFS value lower than -0.50 was 0.88 (95% CI = [0.83, 0.93]).

We additionally studied the relationship between the DGFS and the patient and graft survival. The patients with a DGFS value between -0.5 and 1.2 had a prognosis not significantly different to that of patients with a DGFS lower than -0.5 (HR = 1.41, p=0.3001).

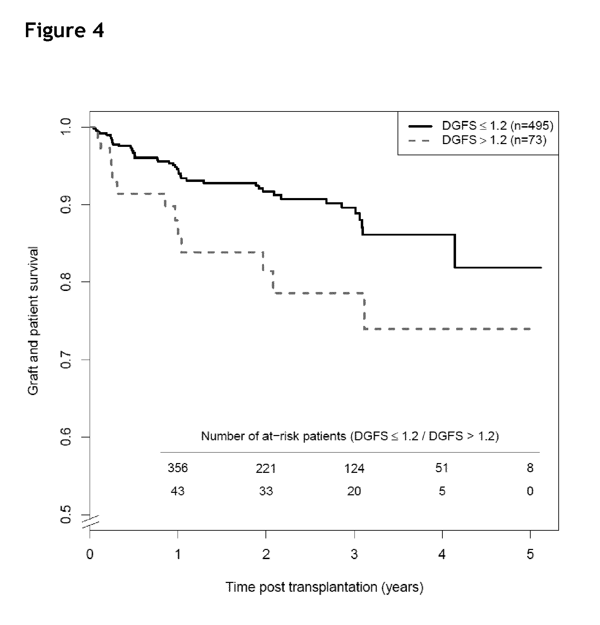

Figure 4 illustrates the survival of patients regarding the threshold at 1.2. Patients with a DGFS higher than 1.2 had a 2.17-fold higher risk of graft failure compared to patients with a lower DGFS value (p=0.0152).

### Deciphering potential bias relating to the integration of the induction therapy in the DGFS

A major bias of our results could be the estimation of the relationship between the induction therapy (ATG or not) and the DGF probability. As shown in tables S2 and S3, patients' characteristics were significantly different when we compared patients with and without ATG treatment. The dialysis duration before transplantation (p=0.0050) and the CIT (p=0.0016) seemed higher for patients who received ATG induction. In addition, the following characteristics were more frequent for patients with ATG induction: female recipient, re-transplantation, past history of hypertension, detectable PRA on class I and II and kidney disease with risk of recurrence. Regarding this description and as expected, induction by ATG was preferentially prescribed for patients at risk of acute rejection and/or DGF. Therefore, the effect of ATG treatment presented in table 3 (OR=1.70) may be underestimated.

**Table S2. Recipient, donor and kidney transplant continuous characteristics (number of missing values, mean and standard deviation) at time of transplantation according to induction therapy.**

| | **Global** | | | **Depleting** | | **No depleting** | | **p-value** |
|---|---|---|---|---|---|---|---|---|
| | **missing** | **mean** | **SD** | **mean** | **SD** | **mean** | **SD** | |
| **Cold ischemia time** (hours) | 0 | 19.21 | 7.02 | 19.78 | 7.13 | 18.51 | 6.81 | 0.0016 |
| **Recipient age** (years) | 0 | 51.92 | 13.19 | 51.53 | 12.96 | 52.4 | 13.47 | 0.2509 |
| **Donor age** (years) | 2 | 51.91 | 15.6 | 51.17 | 15.48 | 52.83 | 15.72 | 0.0625 |
| **BMI** (kg/m²) | 9 | 24.07 | 4.44 | 23.93 | 4.54 | 24.24 | 4.3 | 0.2197 |
| **Donor serum creat.** (µlmol/l) | 5 | 91.02 | 60.67 | 90.05 | 50.76 | 92.2 | 71.02 | 0.5496 |
| **Duration in dialysis** (years) | 3 | 4.07 | 3.92 | 4.35 | 4.35 | 3.74 | 3.27 | 0.0050 |

**Table S3. Recipient, donor and kidney transplant qualitative characteristics (number of missing values, mean and standard deviation) at time of transplantation according to induction therapy.**

| | **Global** | | | **Depleting** | | **No depleting** | | **p-value** |
|---|---|---|---|---|---|---|---|---|
| | **missing** | **N** | **%** | **N** | **%** | **N** | **%** | |
| **Recipient gender** | 0 | | | | | | | 0.0031 |
| male | | 748 | 60.4 | 388 | 56.7 | 360 | 65 | |
| female | | 490 | 39.6 | 296 | 43.3 | 194 | 35 | |
| **Donor gender** | 9 | | | | | | | 0.5772 |
| male | | 720 | 58.6 | 393 | 57.9 | 327 | 59.5 | |
| female | | 509 | 41.4 | 286 | 42.1 | 223 | 40.5 | |
| **Rank of the transplantation** | 0 | | | | | | | 0.0001 |
| first | | 927 | 74.9 | 424 | 62 | 503 | 90.8 | |
| second | | 255 | 20.6 | 208 | 30.4 | 47 | 8.5 | |
| third or more | | 56 | 4.5 | 52 | 7.6 | 4 | 0.7 | |
| **Donor cause of death** | 3 | | | | | | | 0.2831 |
| vascular causes | | 715 | 57.9 | 385 | 56.5 | 330 | 59.6 | |
| others causes | | 520 | 42.1 | 296 | 43.5 | 224 | 40.4 | |
| **History of diabetes** | 0 | | | | | | | 0.4152 |
| yes | | 157 | 12.7 | 82 | 12 | 75 | 13.5 | |
| no | | 1081 | 87.3 | 602 | 88 | 479 | 86.5 | |
| **History of hypertension** | 0 | | | | | | | 0.0246 |
| yes | | 1000 | 80.8 | 568 | 83 | 432 | 78 | |
| no | | 238 | 19.2 | 116 | 17 | 122 | 22 | |
| **Cardiovascular events** | 0 | | | | | | | 0.8604 |
| yes | | 448 | 36.2 | 249 | 36.4 | 199 | 35.9 | |
| no | | 790 | 63.8 | 435 | 63.6 | 355 | 64.1 | |
| **History of dyslipidemia** | 0 | | | | | | | 0.8203 |
| yes | | 384 | 31 | 214 | 31.3 | 170 | 30.7 | |
| no | | 854 | 69 | 470 | 68.7 | 384 | 69.3 | |
| **History of hepatitis B** | 0 | | | | | | | 0.6035 |
| yes | | 81 | 6.5 | 47 | 6.9 | 34 | 6.1 | |
| no | | 1157 | 93.5 | 637 | 93.1 | 520 | 93.9 | |
| **History of neoplasia** | 0 | | | | | | | 0.3353 |
| yes | | 123 | 9.9 | 73 | 10.7 | 50 | 9 | |
| no | | 1115 | 90.1 | 611 | 89.3 | 504 | 91 | |
| **Anti-class I PRA** | 0 | | | | | | | 0.0001 |
| detectable | | 468 | 37.8 | 331 | 48.4 | 137 | 24.7 | |
| undetectable | | 770 | 62.2 | 353 | 51.6 | 417 | 75.3 | |
| **Anti-class II PRA** | 0 | | | | | | | 0.0001 |
| detectable | | 446 | 36 | 338 | 49.4 | 108 | 19.5 | |
| undetectable | | 792 | 64 | 346 | 50.6 | 446 | 80.5 | |
| **Initial kidney disease** | 2 | | | | | | | 0.0387 |
| with risk of recurrence | | 373 | 30.2 | 223 | 32.6 | 150 | 27.2 | |
| without risk of recurrence | | 863 | 69.8 | 461 | 67.4 | 402 | 72.8 | |
| **HLA Mismatches (A+B+DR)** | 0 | | | | | | | 0.0168 |
| 0 to 4 | | 1105 | 89.3 | 616 | 90.1 | 489 | 88.3 | |
| 5 to 6 | | 133 | 10.7 | 68 | 9.9 | 65 | 11.7 | |

In order to decipher this possible bias, we firstly decided to include into the score all these possible confounding factors. The results of this 'full-adjusted model' are presented in table 4.

**Table 4. Results of the final logistic regression (n = 1219, 19 observations removed because of missing data). Δ log OR represents the relative variation of the regression coefficients between this full model and the 5-variables based model (table 3).**

| | **log OR (±SD)** | **OR** | **p-value** | **Δ log OR** |
|---|---|---|---|---|
| **Cold Ischemia time (hours)** | 0.056 (±0.010) | 1.06 | <0.0001 | -3.94% |
| **Donor age (years)** | 0.016 (±0.005) | 1.02 | 0.0014 | -8.51% |
| **Body Mass Index (kg/m²)** | 0.057 (±0.016) | 1.06 | 0.0004 | 5.75% |
| **Donor creatinine level > 108 µmol/L** | 0.575 (±0.161) | 1.77 | 0.0004 | 1.18% |
| **No ATG** | 0.575 (±0.150) | 1.78 | 0.0001 | 8.42% |
| **Duration in dialysis (years)** | 0.030 (±0.017) | 1.03 | 0.0844 | - |
| **Male Recipient** | 0.132 (±0.147) | 1.14 | 0.0369 | - |
| **Rank of the kidney transplantation > 1** | -0.265 (±0.209) | 0.77 | 0.2041 | - |
| **History of hypertension** | -0.216 (±0.175) | 0.81 | 0.2181 | - |
| **Detectable anti-class I PRA** | 0.191 (±0.183) | 1.21 | 0.2966 | - |
| **Detectable anti-class II PRA** | 0.271 (±0.194) | 1.31 | 0.1630 | - |

By considering the same characteristic at baseline (CIT, donor age, BMI, donor creatinine, duration in dialysis, male recipient, graft rank, hypertension and PRA), the estimated benefit of induction therapy with ATG was unchanged (OR=1.78) in this full-adjusted model.

Moreover, by using the validation sample (n=597, nine observations removed due to missing value), the AUC of the full-adjusted model was 0.71 (95% CI = [0.66, 0.76]), which illustrates the uselessness of this full-adjusted model in terms of predictive capacities compared to the 5-variables DGFS (AUC = 0.73, 95% CI = [0.68, 0.77]).

Secondly, we decided to perform a pairwise analysis. Among the 675 patients who received ATG for induction therapy and the 547 recipients without ATG in the training sample, we identified 121 pairs with the same characteristics for variables listed in the table 4 (for continuous variables as donor age, CIT, recipient BMI and duration in dialysis; three classes were defined to obtain balanced effectiveness). The estimated benefit of induction therapy with ATG was similar (OR=1.66) in this pairwise analysis compared the DGFS (OR=1.70).

Finally, the estimated benefit of induction by ATG obtained from our observational cohort from several approaches (OR=1.70 for the DGFS, OR=1.78 for the full-adjusted model, OR=1.66 for the pairwise analysis) were similar to the results obtained recently published by Noel et al. (26) in a randomised trials comparing anti IL2R (n=114, 44.6% of DGF) versus ATG (n=113, 31.5% of DGF). Indeed, based on these data, one can estimate that the risk of DGF was 1.73-fold reduced for patients with ATG (OR=1.73, 95% CI = [1.01, 2.97])

### Discussion

The prediction of the DGF occurrence is important in preventing its deleterious consequences on long-term graft outcome and to improve patient management. Recently, Irish et al. (20) developed a scoring system based on variables collected at the time of transplantation (AUC=0.70). This score has also been validated in North American and European cohorts of patients (27-29). Unfortunately, this tool is based on 18 parameters, probably leading to a limitation of its usefulness in current practices. For this reason, Jeldres et al. proposed a simpler score (six parameters) but from non-recent transplants (since 1976) performed in a single center. Moreover, the two scoring systems did not take into account induction therapy, which remains one of the possible interventions available clinically to potentially decrease the occurrence of DGF. Whilst the benefit of depleting therapy for reducing the risk of DGF is still under debate, our present paper offers additional supportive epidemiologic data obtained in a large cohort from a multicentre database.

In our prospective, multicentric and observational study, we presented a simple score, tentatively called DGFS. Our study was performed on a large cohort of patients gathering 1/3 of all renal transplants performed in France between January 2007 and December 2011. The other inclusion/exclusion criteria are comparable with the study by Irish et al. DGF incidence in our study was 25.4%, very close to published data for cadaveric donor transplants, and to Irish's study with an incidence of 25.7%. The DGFS appeared as predictive as the results presented by Irish et at (validation sample, AUC = 0.73, 95% CI = [0.68, 0.77]). Nevertheless, the DGFS is only based on five parameters, mainly due to the selection procedure based on the contribution to the AUC, not only based on the p-value. Indeed, a risk factor may be significant (p<0.05) without adding value in terms of prediction (30, 31). The five variables, easily available at the time of transplantation, were CIT, recipient BMI, donor age, last donor serum creatinine and use of ATG. The DGFS outperformed the six-parameter scoring system proposed by Jeldres et al., which appeared as predictive as the CIT alone (AUC = 0.64). This result is consistent with other previous studies (6, 32), which demonstrated that CIT appeared to be the most significant risk factor of DGF. Naturally, it suggests that CIT reduction is the main solution to reduce the DGFS.

It is notable that we were not able to perform a direct comparison between the DGFS and Irish's model. Indeed, we did not have all of the necessary parameters. In particular, the donor weight is difficult to evaluate for deceased donors, warm ischemia time is not systemically measured by surgeons, and the donor ethnicity cannot be collected due to French Law. This illustrates the difficulty of using the scoring system proposed by Irish et al.

In contrast, we used additional parameters which have already been demonstrated to be correlated with the DGF risk: induction therapy (26, 33), donor treatment (6), or the precise history of recipients (hepatitis, neoplasia, cardiovascular, etc.).

Beyond the predictive accuracy of our score, it is interesting to demonstrate that induction therapy with ATG was correlated with the DGF risk regardless the value of CIT and other confounding factors. ATG has already been shown in recent studies to minimize DGF risk (26, 33). In a recent randomized trial of 227 high-risk HLA-sensitized kidney transplant patients, Noel et al. (26) reported a DGF prevalence of 31.5% in the ATG group versus 44.6% with antiinterleukin 2-receptor antibody (daclizumab) treatment. In this study, the risk of DGF was therefore 1.73-fold reduced for patients with ATG (OR=1.73, 95% CI = [1.01, 2.97]). This previous randomized trial confirms the reduction of the DGF risk related to the ATG induction therapy we estimated in the DGFS (OR = 1.70, 95% CI = [1.30, 2.23]). This comparison is important, since our observational study may be associated to confounding effects related to indication bias of depleting induction, in contrast to randomized trials. For this reason, we also validated our results by two other statistical approaches in order to ensure the comparability of patients with and without depleting induction therapy.

Equivalent reductions in the risk of DGF for patients with depleting treatment were obtained (OR = 1.78 in the full-adjusted model and OR = 1.66 in a complete pairwise analysis).

Polyclonal anti-thymocyte globulins limit leucocyte activation and renal graft infiltration and the cytokine release, key mechanisms that participate in renal graft ischemia-reperfusion injury and contribute to tissue damage and early organ dysfunction (34-36). Moreover, the use of polyclonal anti-thymocyte antibody allows the possibility to delay the introduction of calcineurin inhibitors after renal transplantation, immunosuppressive maintenance therapy known to have microvascular hemodynamic properties and therefore likely to have an impact on immediate graft function (37). In our study, we show that patients who received anti-thymocyte antibody presented a similar lower risk of DGF regardless of the time of introduction of the CNI inhibitors. Nevertheless, other studies also demonstrated no significant effect of the kind of induction therapy on DGF occurrence, and is likely explained by differences of targeted population studied (16, 38-40).

In contrast to other scoring systems, because the DGFS includes the induction therapy metrics, it may be a useful tool for clinicians to assist in medical decision making to decrease the risk of DGF by using a depleting induction therapy. This should increase the benefit of such treatment in this specific population at-risk of DGF, while its effect is always under debate, probably due to the heterogeneity of highly variable decision making between countries, between centers and even amongst clinicians. As an example, the calculation of the DGFS can reveal that a patient with a BMI at 23 kg/m², who receives a 40-year-old kidney with a good creatinemia at 90 µmol/L and a short CIT at 12 hours will have a 16% chance of presenting a DGF under an induction by anti IL2R or with no induction compared to 10% for to the same patient with ATG induction therapy. In the first case, the risk of DGF is very low and the benefit of an ATG induction therapy is probably irrelevant regarding the corresponding adverse events. In contrast, a patient with a BMI at 28 kg/m², who receives a 53-year-old kidney with a good creatinemia at 90 µmol/L and a medium CIT at 23 hours will have a 36% chance of presenting a DGF under an induction by anti IL2R or with no induction compared to 25% for to the same patient with ATG induction therapy. In this second case, with donor not classified in ECD but with a medium CIT, the decision about the induction therapy may vary importantly between countries/centers/physicians. Therefore, it could be useful for the physician to benefit from such a tool to help the therapeutic decision making.

A 36% chance of DGF may justify the possible adverse event of ATG in order to decrease the risk of DGF by 11%. When the associated applications are available online at www.divat.fr/en/softwares (available on smartphones, tablets or computers); the DGFS will be easily used in routine clinical practice.

We also proposed additional cut-offs for medical decision making based on predictive values. A patient with a DGFS < -0.50 is predicted to have no DGF, with an 88% chance of accuracy. Therefore, this test identifies patients who will not experience a DGF after kidney transplantation with a low false negative rate (12%). This information could be useful for the medical team in order to avoid unnecessary booking dialysis and promote early hospital discharges. In contrast, among patients with a DGFS > 1.2 (observed in 1 /10 of recipients), half of them will experience a DGF. These high-risk patients should be even more closely monitored. For instance their management could be promoted to include the use of biocompatible membranes for dialysis (41), the prescription of calcium inhibitors (42) or depleting treatment at the surgery time. The use of pulsatile perfusion machines could be another option to reduce DGF risk and improve graft survival compared to simple cold storage as recently described (43).

The management of these high-risk patients is even more important since we also demonstrated the worst graft and patient survival for this group (HR=2.17, p=0.0152). These results are not surprising knowing the detrimental clinical effect of DGF on grafts and patients survival, which are well documented in literature (4, 5). In addition to being independently associated with an increased risk for graft failure, patients with DGF were significantly more likely to die with a functioning graft and from cardiovascular and infectious deaths compared with patients without DGF (4, 44).

Among the five parameters computed in the DGFS, it is important to highlight donor age and the last donor serum creatinine measurement. Indeed, DGF incidence has not dramatically changed over the past 10 years, likely resulting from increased well-established donor risk factors such as age and associated comorbidities, i.e. the increased frequency of expanded criteria donors and the non heart-beating donors. Beside these two donor parameters, the CIT and the induction therapy, the fifth parameter is the recipient BMI. The DGFS increased linearly with the BMI level (OR = 1.06, p = 0.0004). BMI was also included in Irish's nomogram, while Jeldres et al. considered the recipient weight in their model. The overweight parameter is of a recent interest according to the increased obese candidates for kidney transplantation (45, 46). In this context, Weissenbacher et al. (47) closely studied the impact of recipient and donor BMI on DGF among a cohort of 1113 deceased donor kidney transplant patients. They also observed that recipient BMI was independently associated with DGF in multivariate analysis. They suggested that this result could be the consequence of longer CIT (due to longer vessel preparation time) and anastomosis time (due to impaired vascular access). Another hypothesis could be that obesity is characterized by a proinflammatory environment with a lack of antiinflammatory mediators that might interfere with the cellular and molecular mechanisms involved in ischemia-reperfusion injuries (48, 49).

In conclusion, we developed a score to predict DGF. Regardless the good predictive capacity of the DGFS, this type of scoring system is never perfect. Nevertheless, it may, for the first time, constitute a useful tool for clinicians in practice. Therapeutic strategies can; for instance, be adapted regarding this risk stratification. To ensure its utility, the DGFS is based on small number of easily available parameters. It includes induction therapy characteristics which possibly represent one of the major tools for clinician to decrease the DGF risk, and finally this application is available from electronic devices.

### References

1. Miller J, Mendez R, Pirsch JD, Jensik SC. Safety and efficacy of tacrolimus in combination with mycophenolate mofetil (MMF) in cadaveric renal transplant recipients. FK506/MMF Dose-Ranging Kidney Transplant Study Group. Transplantation 2000; 69: 875-880.
2. Yarlagadda SG, Coca SG, Garg AX, Doshi M, et al. Marked variation in the definition and diagnosis of delayed graft function: a systematic review. Nephrol Dial Transplant 2008; 23: 2995-3003.
3. Perico N, Cattaneo D, Sayegh MH, Remuzzi G. Delayed graft function in kidney transplantation. Lancet 2004; 364: 1814-1827.
4. Tapiawala SN, Tinckam KJ, Cardella CJ, Schiff J, et al. Delayed graft function and the risk for death with a functioning graft. J Am Soc Nephrol 2010; 21: 153-161.
5. Yarlagadda SG, Coca SG, Formica RN, Jr., Poggio ED, et al. Association between delayed graft function and allograft and patient survival: a systematic review and meta-analysis. Nephrol Dial Transplant 2009; 24: 1039-1047.
6. Giral M, Bertola JP, Foucher Y, Villers D, et al. Effect of brain-dead donor resuscitation on delayed graft function: results of a monocentric analysis. Transplantation 2007; 83: 1174-1181.
7. Schnuelle P, Gottmann U, Hoeger S, Boesebeck D, et al. Effects of donor pretreatment with dopamine on graft function after kidney transplantation: a randomized controlled trial. JAMA 2009; 302: 1067-1075.
8. Arumugam TV, Shiels IA, Strachan AJ, Abbenante G, et al. A small molecule C5a receptor antagonist protects kidneys from ischemia/reperfusion injury in rats. Kidney Int 2003; 63: 134-142.
9. Kelly KJ, Williams WW, Jr., Colvin RB, Bonventre JV. Antibody to intercellular adhesion molecule 1 protects the kidney against ischemic injury. Proc Natl Acad Sci U S A 1994; 91: 812-816.
10. Knight SF, Kundu K, Joseph G, Dikalov S, et al. Folate receptor-targeted antioxidant therapy ameliorates renal ischemia-reperfusion injury. J Am Soc Nephrol 2012; 23: 793-800.
11. Thurman JM, Royer PA, Ljubanovic D, Dursun B, et al. Treatment with an inhibitory monoclonal antibody to mouse factor B protects mice from induction of apoptosis and renal ischemia/reperfusion injury. J Am Soc Nephrol 2006; 17: 707-715.
12. Kaden J, May G, Strobelt V, Groth J, et al. Intraoperative T-cell depletion prior to completion of anastomoses by high-dose single ATG bolus as a new approach to improve long-term results after kidney transplantation. Transplant Proc 1997; 29: 344-347.
13. Kyllonen LE, Eklund BH, Pesonen EJ, Salmela KT. Single bolus antithymocyte globulin versus basiliximab induction in kidney transplantation with cyclosporine triple immunosuppression: efficacy and safety. Transplantation 2007; 84: 75-82.
14. Turunen AJ, Lindgren L, Salmela KT, Kyllonen LE, et al. Association of graft neutrophil sequestration with delayed graft function in clinical renal transplantation. Transplantation 2004; 77: 1821-1826.
15. Haririan A, Morawski K, Sillix DH, El-Amm JM, et al. Induction therapy with basiliximab versus Thymoglobulin in African-American kidney transplant recipients. Transplantation 2005; 79: 716-721.
16. Mourad G, Rostaing L, Legendre C, Garrigue V, et al. Sequential protocols using basiliximab versus antithymocyte globulins in renal-transplant patients receiving mycophenolate mofetil and steroids. Transplantation 2004; 78: 584-590.
17. Sollinger H, Kaplan B, Pescovitz MD, Philosophe B, et al. Basiliximab versus antithymocyte globulin for prevention of acute renal allograft rejection. Transplantation 2001; 72: 1915-1919.
18. Ulrich F, Niedzwiecki S, Pascher A, Kohler S, et al. Long-term outcome of ATG vs. Basiliximab induction. Eur J Clan Invest 2011; 41: 971-978.
19. Irish WD, McCollum DA, Tesi RJ, Owen AB, et al. Nomogram for predicting the likelihood of delayed graft function in adult cadaveric renal transplant recipients. J Am Soc Nephrol 2003; 14: 2967-2974.
20. Irish WD, Ilsley JN, Schnitzler MA, Feng S, et al. A risk prediction model for delayed graft function in the current era of deceased donor renal transplantation. Am J Transplant 2010; 10: 2279-2286.
21. Fleiner F, Budde K, Dragun D, Hartmann M, et al. Differences in reporting of acute rejections between American and European publications of large immunosuppressive trials impair comparability of study results. Transplant Proc 2005; 37: 2048-2050.
22. Pallet N, Thervet E, Alberti C, Emal-Aglae V, et al. Kidney transplant in black recipients: are African Europeans different from African Americans? Am J Transplant 2005; 5: 2682-2687.
23. Jeldres C, Cardinal H, Duclos A, Shariat SF, et al. Prediction of delayed graft function after renal transplantation. Can Urol Assoc J 2009; 3: 377-382.
24. R Development Core Team. R: A Language and Environment for Statistical Computing: Vienna, Austria, 2010.
25. Irwin RJ, Irwin TC. A principled approach to setting optimal diagnostic thresholds: where ROC and indifference curves meet. Eur J Intern Med 2011; 22: 230-234.
26. Noel C, Abramowicz D, Durand D, Mourad G, et al. Daclizumab versus antithymocyte globulin in high-immunological-risk renal transplant recipients. J Am Soc Nephrol 2009; 20: 1385-1392.
27. Moore J, Ramakrishna S, Tan K, Cockwell P, et al. Identification of the optimal donor quality scoring system and measure of early renal function in kidney transplantation. Transplantation 2009; 87: 578-586.
28. Rodrigo E, Minambres E, Ruiz JC, Ballesteros A, et al. Prediction of delayed graft function by means of a novel web-based calculator: a single-center experience. Am J Transplant 2012; 12: 240-244.
29. Tiong HY, Goldfarb DA, Kattan MW, Alster JM, et al. Nomograms for predicting graft function and survival in living donor kidney transplantation based on the UNOS Registry. J Urol 2009; 181: 1248-1255.
30. Foucher Y, Combescure C, Ashton-Chess J, Giral M. Prognostic markers: data misinterpretation often leads to overoptimistic conclusions. Am J Transplant 2012; 12: 1060-1061.
31. Ware JH. The limitations of risk factors as prognostic tools. N Engl J Med 2006; 355: 2615-2617.
32. Quiroga I, McShane P, Koo DD, Gray D, et al. Major effects of delayed graft function and cold ischaemia time on renal allograft survival. Nephrol Dial Transplant 2006; 21: 1689-1696.
33. Mourad G, Morelon E, Noel C, Glotz D, et al. The role of Thymoglobulin induction in kidney transplantation: an update. Clan Transplant 2012; 26: E450-464.
34. Boros P, Bromberg JS. New cellular and molecular immune pathways in ischemia/reperfusion injury. Am J Transplant 2006; 6: 652-658.
35. Huang Y, Rabb H, Womer KL. Ischemia-reperfusion and immediate T cell responses. Cell Immunol 2007; 248: 4-11.
36. Siedlecki A, Irish W, Brennan DC. Delayed graft function in the kidney transplant. Am J Transplant 2011; 11: 2279-2296.
37. Kamar N, Garrigue V, Karras A, Mourad G, et al. Impact of early or delayed cyclosporine on delayed graft function in renal transplant recipients: a randomized, multicenter study. Am J Transplant 2006; 6: 1042-1048.
38. Abou-Ayache R, Buchler M, Lepogamp P, Westeel PF, et al. CMV infections after two doses of daclizumab versus thymoglobulin in renal transplant patients receiving mycophenolate mofetil, steroids and delayed cyclosporine A. Nephrol Dial Transplant 2008; 23: 2024-2032.
39. Ciancio G, Burke GW, Gaynor JJ, Carreno MR, et al. A randomized trial of three renal transplant induction antibodies: early comparison of tacrolimus, mycophenolate mofetil, and steroid dosing, and newer immune-monitoring. Transplantation 2005; 80: 457-465.
40. Lebranchu Y, Bridoux F, Buchler M, Le Meur Y, et al. Immunoprophylaxis with basiliximab compared with antithymocyte globulin in renal transplant patients receiving MMF-containing triple therapy. Am J Transplant 2002; 2: 48-56.
41. Woo YM, Craig AM, King BB, Junor BJ, et al. Biocompatible membranes do not promote graft recovery following cadaveric renal transplantation. Clan Nephrol 2002; 57: 38-44.
42. Neumayer HH, Kunzendorf U, Schreiber M. Protective effects of calcium antagonists in human renal transplantation. Kidney Int Suppl 1992; 36: S87-93.
43. Moers C, Smits JM, Maathuis MH, Treckmann J, et al. Machine perfusion or cold storage in deceased-donor kidney transplantation. N Engl J Med 2009; 360: 7-19.
44. Patel SJ, Duhart BT, Jr., Krauss AG, Moore LW, et al. Risk factors and consequences of delayed graft function in deceased donor renal transplant patients receiving antithymocyte globulin induction. Transplantation 2008; 86: 313-320.
45. Abbott KC, Glanton CW, Agodoa LY. Body mass index and enrollment on the renal transplant waiting list in the United States. J Nephrol 2003; 16: 40-48.
46. Friedman AN, Miskulin DC, Rosenberg IH, Levey AS. Demographics and trends in overweight and obesity in patients at time of kidney transplantation. Am J Kidney Dis 2003; 41: 480-487.
47. Weissenbacher A, Jara M, Ulmer H, Biebl M, et al. Recipient and donor body mass index as important risk factors for delayed kidney graft function. Transplantation 2012; 93: 524-529.
48. Okamoto Y, Christen T, Shimizu K, Asano K, et al. Adiponectin inhibits allograft rejection in murine cardiac transplantation. Transplantation 2009; 88: 879-883.
49. Vgontzas AN, Bixler EO, Papanicolaou DA, Chrousos GP. Chronic systemic inflammation in overweight and obese adults. JAMA 2000; 283: 2235; author reply 2236.
50. Peake & Whiting, Measurement of Serum Creatinine - Current Status and Future Goals Clin Biochem Rev, 2006, 27(4): 173-184.
51. Bishop et al., Approaching the Promise of Operational Tolerance in Clinical Transplantation. Transplantation, 2011, 91(10): 1065-1074.
52. Kovarik, From Immunosuppression to Immunomodulation: Current Principles and Future Strategies. Pathobiology, 2013, 80: 275-281.
53. Mourad et al., The role of Thymoglobulin induction in kidney transplantation: an update. Clin Transplant, 2012, 26(5): E450-464.
54. Szumilas, Explaining Odds Ratios. J Can Acad Child Adolesc Psychiatry, 2010, 19(3): 227-230.

## Claims

1. A method for predicting delayed graft function (DGF) after kidney transplantation in patient, said method comprising the steps of:
a) determining the following parameters:
• Induction therapy,
• Cold ischemia time (CIT),
• Donor creatinin levels,
• Donor age, and
• Patient BMI;
b) calculating a score (DGFS) by computing the parameters of step a), and
c) determining the risk of DGF in view of the DGFS.

2. The method of claim 1, wherein the induction therapy consists of an Anti Thymocyte Globulin (ATG) therapy.

3. The method of any one of claims 1 or 2,the calculation of the DGFS comprises the steps of:
i) multiplying the logarithm of the odds ratio and the values of the explicative variables,
ii) summing up the 5 numbers obtained in i),
iii) subtracting 3.546 to the numbers obtained in iii),
iv) dividing the number obtained in iii) by 0.626.

4. The method of any one of claims 1 to 3, wherein the risk of DGF is low if the DGFS is lower than -0.50.

5. The method of any one of claims 1 to 4, wherein the risk of the DGF is high if the DGFS is higher than 1.20.

6. A method for assessing the need of a DGF therapy for a kidney transplantation, said method comprising the steps of:
a) determining the risk of DGF according to the method of any one of claims 1 to 5, and
b) administering a therapy after the kidney transplantation if the DGFS is above a threshold.

7. The method of claim 1, wherein the said therapy consists of an Anti Thymocyte Globulin (ATG) therapy.

8. The method of any one of claims 1 or 7, wherein no therapy is administered if the DGFS is DGFS is lower than -0.50.

9. The method of any one of claims 1 or 7, wherein the said therapy is administered if the DGFS is higher than 1.20.

10. The method of any one of claims 1 or 7, wherein comprises the steps of:
a) determining the risk of DGF for the patient with ATG induction according to the method of the invention,
b) determining the risk of DGF for the said patient with no induction or with an induction by an IL2R antibody according to the method of the invention, and
c) administering a therapy if the DGFS calculated in step a) is inferior to the DGFS calculated in step b).

11. A product/computer program containing a set of instructions characteristic of implementation of the method of any one of claims 1 to 5.

12. A processing system including a computation unit and an input interface, said system including means for implementing the method of any one of claims 1 to 5.
